# EUROPEAN PATENT APPLICATION

(11) **EP 1 988 175 A1**
(43) Date of publication of application: **05.11.2008**
(21) Application number: 07090093.1
(22) Date of filing: 03.05.2007
(51) Int. Cl.: C12Q 1/68

(54) **Predictive RNA profiles**

(71) Applicant: Bayer Schering Pharma Aktiengesellschaft, 13353 Berlin (DE)
(72) Inventor: Koczan, Dirk, 18057 Rostock (DE); Zettl, Uwe Kai, 18057 Rostock (DE); Goertsches, Robert, 18057 Rostock (DE); Möller, Steffen, 18057 Rostock (DE); Serrano-Fernándes, Pablo, 70115 Szcecin (PL)

(57) **Abstract**

Multiple sclerosis (MS) is an autoimmune disease of the central nervous system. The present invention is directed to predictive RNA profiles and their use for predicting the success in modulation of a disease course in a patient treated with Interferon beta 1b (IFNβ1b) *ex vivo* as well as to certain genes which are specifically expressed during the course of the disease.

## Description

The present invention is directed to predictive RNA profiles and their use for predicting the success in modulation of a disease course *ex vivo* as well as to certain genes which are specifically expressed during the course of the disease.

Multiple sclerosis (MS) is the most prevalent chronic autoimmune, neurodegenerative disorder of the central nervous system (CNS). Despite substantial progress, treatment of MS and other autoimmune diseases is only moderately effective. It is anticipated that the treatment of autoimmune diseases with single drugs or biological approaches will in the future be complemented, or even replaced, by combination therapies, which include immunomodulation, elimination of infectious triggers and tissue repair.

In the attempt to identify a gene expression profile characteristic for MS, several expression studies have been published in the last years. In all these studies, the authors investigated the gene expression pattern of peripheral blood mononuclear cells (PBMC) from relapsing remitting Multiple sclerosis (RRMS) patients, generally using cDNA microarrays (Ramanathan M., Weinstock-Guttman B, Nguyen LT, et al. In vivo gene expression revealed by cDNA arrays: the pattern in relapsing-remitting multiple sclerosis patients compared with normal subjects. J Neuroimmunol. 2001; 116:213-219; Maas K, Chan S. Parker J, et al., Cutting edge: molecular portrait of human autoimmune disease. J Immunol. 2002;169:5-9; Bromprezzi R, Ringner M, Kim S, et al. Gene expression profile in multiple sclerosis patients and healthy controls: identifying pathways relevant to disease. Hum Mol Genet. 2003;12:2191-2199) or DNA microarrays (Achiron A, Gurevich M, Freidman N, Kaminski N, Mandel M. Blood transcriptional signatures of multiple sclerosis: unique gene expression of disease activity. Ann Neurol. 2004;55:410-417; Iglesias AH, Camelo S, Hwang D, Villanueva R, Stephanopoulos G, Dangong F. Microarray detection of E2F pathway activation and other targets in multiple sclerosis peripheral blood mononuclear cells. J Neuroimmunol. 2004;150:163-177).

Especially cDNA microarray driven analyses facilitate medical research by documenting detailed responses of cells and tissues to both disease and the intended and unintended effects of drug treatments (Goertsches R, Serrano-Fernandez P, Moller S, Koczan D, Zettl UK. Multiple sclerosis therapy monitoring based on gene expression. Curr Pharm Des. 2006;12(29):3761-79).

Despite the above mentioned attempts to provide markers that aid in the diagnosis, prognosis, and/or monitoring of MS, the set of presently available markers still is insufficient.

Besides the need for reliable surrogate biomarkers the need for simple prognostic models, which help to predict the outcome of a disease modifying treatment is also extremely demanding.

It is therefore an object of the present invention, to provide a simple prognostic model to predict the clinical outcome of treatment of a disease, in particular MS.

The problem is solved by combination of several aspects of patient data. It was found that a systematic time-dependent gene expression analysis from Interferon beta 1b (IFNβ1b) treated relapsing remitting Multiple sclerosis (RRMS) patients delivers sets of genes (classifier) that provide a rational basis for predicting the success in modulation of the disease course.
Such a method for the diagnosis and prediction of the success in modulating of the disease course in a patient, comprises a) providing a biological sample from an IFNβ1b treated RRMS patients, b) providing a biological sample from a healthy donor or a reference value indicative for a healthy donor or a non treated MS patient, c) determining the level of expression of one or more genes according to table 1, and d) whereby the upregulation of one or more selected genes of table 1 is indicative for the successful course of the treatment.
The method used for gene selection in building the prognostic classifier is a simple decision (or prognostic) tree, adapted from classification trees (Briemann L, Friedman JH, Olshen RA, Stone CJ. Classification and regression trees. Belmont, CA: Wadsworth International, 1984). It is based on filtering tools (principal component analysis, k-means) that preselected clustered genes from the set of differentially expressed genes (increased/ decreased).
These clustered genes can then be tested for their clinically relevant value, if they correlated with the outcome classes "response" or "non-response" to IFNβ-1 b treatment.
Attained decision trees were tested by evaluating specified classifiers on the test set, thereby estimating the prediction accuracy (sensitivity and specificity).
Different classifiers yield different prediction accuracies in settings with different prevalence for the outcome categories such as "relapse" and "disability progression".

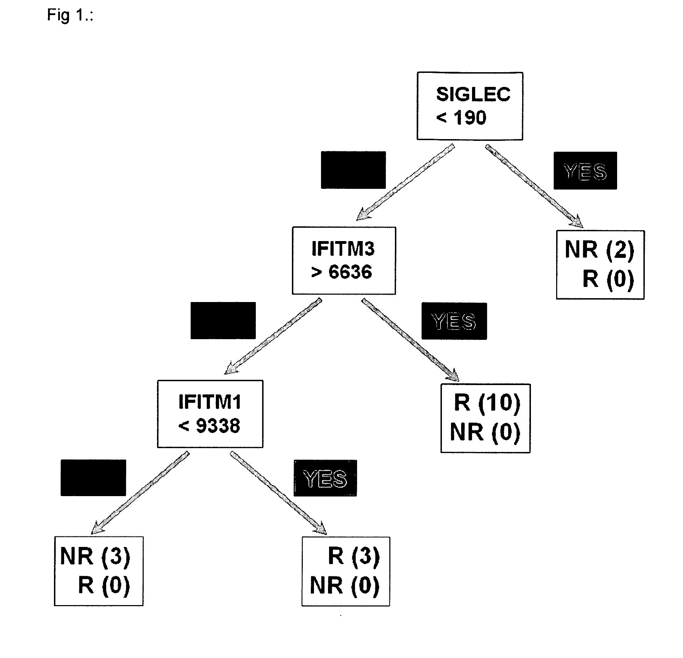

The decision/prognostic trees contained in total a set of 17 genes (tab. 1) that performed reasonably well with a good outcome at 48h, 1 month or 1 year into therapy.
For each time point and subcategory, the tree analysis provided a tree structure, with 2 branches at each step of the tree and new branches being formed until the data had no further important prognostic information, meaning that the data could not be further subdivided by any of the possible prognostic factors to be of any benefit in predicting a clinical response) (fig. 1). The clinical parameters (relapse rate, disability progression, clinical score) correlated differently well with attained molecular RNA profiles at different times. Best results were found for one month into therapy and the clinical parameter "disability progression".
Within each of these subgroups (time point) different sets of prognostic RNA factors were identified, although in three cases two probe sets coding each for TRAIL, SIGLEC1 and RSAD2 recurrently appeared.

Supervised prediction uses both the outcome information and the expression data to develop and evaluate a classifier that can be used to predict outcome in new patients, based only on their expression profiles.

Subject matter of the present invention is the use of the identified genes to predict the success in modulation of a disease course in a patient treated with Interferon beta 1b (IFNβ1b) *ex vivo* whereas the disease can be elected out of a group comprising multiple sclerosis (MS), diabetes mellitus type 1 (IDDM), systemic lupus erythematosus (SLE), Sjögren's syndrome, Hashimoto's thyroiditis, Graves' disease, Crohn's Disease, and rheumatoid arthritis (RA).

Another embodiment of the present invention is a method for the diagnosis and prediction of the success in modulating of the disease course in a patient according to the present invention comprising a) providing a biological sample from an Interferon beta 1b (IFNβ1b) treated relapsing remitting Multiple sclerosis (RRMS) patients, b) providing a biological sample from a healthy donor or a reference value indicative for a healthy donor or a non treated MS patient, c) determining the level of expression of one or more genes according to table 1, and d) whereby the upregulation of one or more genes of table 1 is indicative for the successful course of the treatment.

In yet another preferred embodiment thereof, the *in vitro* method for diagnosis and prediction of the success in modulating of the disease course in a patient as described above comprises determining the genes according to table 1, comprising microarray analysis, real-time PCR and/or flow cytometry analysis. Other methods for determining gene or protein expression are known of skill in the art and can be taken from the respective literature, these can also be applied, such as antigen-based tests.

In another embodiment thereof, the *in vitro* method for the diagnosis and prediction of the success in modulating of the disease course in a patient as described above is characterized in that said method is performed at least one to four times at 48h, 1 month or 1 year into therapy depending on the particular status of the patient that will be diagnosed and/or monitored.

The marker according to the present invention allows for an improved monitoring of the progression of the disease in patient which are treated with IFNβ1b by the attending physician. For a diagnosis, the expression of the marker can easily be measured based on the blood or serum of a patient followed by measuring of the expression levels by means of microarray technology.
Another aspect of the present invention relates to the use of time-dependent gene expression analysis of the genes according to table 1 from Interferon beta 1b (IFNβ1b) treated relapsing remitting Multiple sclerosis (RRMS) patients for predicting the success in modulation of the disease course. In particular, the *in vitro* methods for diagnosis and/or monitoring of multiple sclerosis in an RRMS patient treated with IFNβ1b herein are characterized in that said methods which are performed at least one to four times a year at 48h, 1 month or 1 year into therapy depending on the particular status of the patient that will be diagnosed and/or monitored.

Another preferred embodiment is related to a kit, comprising suitable material and compounds for performing the methods described above. Such materials are generally known to a person of skill in the art and can be selected from chemicals, buffers, disposable test devices, foil packs, instructions for using said test kit, buffers, antibodies, color charts, reference blood or serum samples from healthy donors, and plastic materials such as dishes and tubes. More preferred is a kit as described above, wherein said kit comprises materials and compounds suitable for a point-of-care analysis. Point-of-care (POC) analysis is normally defined as testing that is performed outside physical facilities of the clinical laboratory, in proximity to the patient on whom the testing is performed. The central feature of POC testing is that it does not require permanent, dedicated space.

### 1. Methods

### 1.1 Clinical (patients and samples)

Subject of the study was a group of 18 patients (female/male ratio 2:1) with diagnosed relapsing remitting MS (RRMS) according to Poser and McDonald criteria and ages ranging from 21 to 66 years (mean: 42). The patients were prescribed a first immunotherapy with IFNβ1b after the second exacerbation of the disease. 8 MIU of the drug were injected subcutaneously every second day. Peripheral blood of each patient was drawn before onset of therapy (t0; baseline) and subsequently two days (t1), one month (t2), one year (t3) and two years (t4) after initiation (Goertsches et al., 2006). Samples were taken and immediately processed at same time of the day; always 42h after the last injection of IFNβ1b and prior to upcoming IFNβ1b injection. A second independent cohort of RRMS patients (n=7) was included and treated as described. Both groups were classified by stringent clinical criteria as IFNβ-1b responders or non-responders after 2 years follow-up.

### 1.2 Technical

Peripheral blood mononuclear cells (PBMCs) of IFNβ1b (Betaferon®) treated MS patients were isolated by centrifugation on a Ficoll density gradient and subsequently mRNA isolated (Qiagen kit). RNA was labeled and analyzed by microarray technology (GeneChip® U133 set, Affymetrix), thereby simultaneously measuring the expression levels of about 45 000 transcripts (probe sets). Quality control and normalization procedures were performed according to Affymetrix manual. The expression level and corresponding accuracy detection p-value of all transcripts were calculated applying the MAS 5 algorithm (Affymetrix). Signal values per individual were then compared with baseline levels in order to identify those transcripts, which were differentially expressed after therapy onset over the 95% significance threshold (Wilcoxon signed rank paired test; p<0.05). Transcripts which passed that threshold in at least one of the samples were considered for further analysis (e.g. receiving a "Present" call for at least one patient at a particular time point).

### 1.3 Statistical analysis:

In order to distinguish subgroups of treated patients based on generated RNA-profiles and clinical parameters, cluster selection and supervised learning algorithms were applied.
Supervised methods utilize the outcome data in developing a classifier. In many studies, most genes are not correlated with outcome. Consequently, the expression profiles as a whole are not effective in predicting outcome because the information in the informative genes is overloaded by the number of noninformative genes. Classifiers based on combining information from the informative genes that are correlated with outcome give more accurate predictions, and supervised methods can identify those genes (Radmacher MD, McShane LM, Simon R. A paradigm for class prediction using gene expression profiles. J Comput Biol. 2002;9(3):505-11).
The test of a classifier is whether it predicts accurately for independent data. Therefore, a fundamental rule is that the samples used for the validation step must not have been used for building the classifier. Two types of methods can be used to ensure that this principle is not violated: the cross-validation procedure and the split-sample procedure. The dual-validation procedure was applied in which the classifier and its validation was achieved both with a cross-validation procedure in the training set of 18 patients and by using an additional independent sample of seven RRMS patients.

### 1.4. Method to create prognostic classifier (training set; n=18)

The method used for gene selection in building the prognostic classifier was simple decision (or prognostic) tree, adapted from classification trees (Briemann L, Friedman JH, Olshen RA, Stone CJ. Classification and regression trees. Belmont, CA: Wadsworth International, 1984). It is based on filtering tools (principal component analysis, k-means) that preselect clustered genes from the set of differentially expressed genes (Increase, Decrease).
These clustered genes were then tested for their clinically relevant value, if they correlated with the outcome classes "response" or "non-response" to IFNβ-1b treatment. Subcategoric binary variables were "relapse: Yes / No", "disability progression: Yes / No" and a combined variable, the "clinical score". The discriminating variables were absolute gene expression value and the ratio between a certain time (t1, t2, t3, t4,) and t0, respectively.

1.5 Methods used for validating the classifier performance (test set; n=7).
Attained decision trees were tested by evaluating specified classifiers on the test set, thereby estimating the prediction accuracy (sensitivity and specificity).
Different classifiers yield different prediction accuracies in settings with different prevalence for the outcome categories such as "relapse" and "disability progression".
The performance of classifiers trained to predict the "response or non-response" binary outcome is presented in a contingency table of these two categories for both true and predicted status.
All the computational steps were performed by the open source computer language software "R" (Bioconductor).

### Figure and Table

Figure 1: Decision tree for variable "disability progression" for INFβ-1b treated RRMS patients; R: Therapy Responder; NR: Therapy Non Responder.

Table 1: Promising biomarker genes of prognostic value for INFβ-1b therapy in RRMS patients.

## Claims

1. Use of the genes according to table 1 to predict the success in modulation of a disease course in a patient treated with Interferon beta 1b (IFNβ1b) *ex vivo* whereas the disease can be elected out of a group comprising multiple sclerosis (MS), diabetes mellitus type 1 (IDDM), systemic lupus erythematosus (SLE), Sjögren's syndrome, Hashimoto's thyroiditis, Graves' disease, Crohn's Disease, and rheumatoid arthritis (RA).

2. *In vitro* method for the diagnosis and prediction of the success in modulating of the disease course in a patient according to the present invention comprising a) providing a biological sample from an Interferon beta 1b (IFNβ1b) treated relapsing remitting Multiple sclerosis (RRMS) patients, b) providing a biological sample from a healthy donor or a reference value indicative for a healthy donor or a non treated MS patient, c) determining the level of expression of one or more genes according to table 1, and d) whereby the upregulation of one or more genes of table 1 is indicative for the successful course of the treatment.

3. Method of claim 2 comprising determining the genes according to table 1, comprising microarray analysis, real-time PCR and/or flow cytometry analysis.

4. Method of claim 2 whereby said method is performed at least one to four times at 48h, 1 month or 1 year into therapy.

5. Use of time-dependent gene expression analysis of the genes according to table 1 from Interferon beta 1b (IFNβ1b) treated relapsing remitting Multiple sclerosis (RRMS) patients for predicting the success in modulation of the disease course.

6. Kit, comprising suitable material and compounds for performing the methods described above, comprising chemicals, buffers, disposable test devices, foil packs, instructions for using said test kit, buffers, antibodies, color charts, reference blood or serum samples from healthy donors, and plastic materials such as dishes and tubes.

7. Kit according to claim 6 wherein said kit comprises materials and compounds suitable for a point-of-care analysis (POC).
**Table 1:**
| | **probe ID** | **Gene** | **Gene name** | **time** | **reg** |
|---|---|---|---|---|---|
| 1 | 44673_at | Sialoadhesin (2 probesets) | SIGLEC1 | d2, m1 m1 | Up |
| | 219519_s_at | | | | |
| 2 | 242625_at; | Radical S-adenosyl methionine domain containin 2 (2 probesets) | RSAD2 | d2 m1 | Up |
| | 213797 at | | | | |
| 3 | 202086_at | Myxovirus (influenza virus) resistance 1 | Mx1 | d2 | Up |
| 4 | 204994_at | Myxovirus (influenza virus) resistance 2 (mouse) | Mx2 | d2 | Up |
| 5 | 202145_at | Lymphocyte antigen 6 complex, locus E | LY6E | d2 | Up |
| 6 | 214453_s_at | Interferon-induced protein 44 | IFI44 | d2 | Up |
| 7 | 229450_at | Sequence located in IFIT-3 (Prom) or LIPA | IFIT-3 Prom | d2 | Up |
| 8 | 226757_at | Interferon-induced protein w tetratricopeptide rep 2 | IFIT2 | d2 | Up |
| 9 | 212203_x_at | Interferon induced transmembrane protein 3 (1-8U) | IFITM3 | d2, m1 | Up |
| 10 | 201601_x_at | Interferon induced transmembrane protein 1 (9-27) | IFITM1 | m1 | Up |
| 11 | 203964_at | N-myc (and STAT) interactor | NMI | m1 | Up |
| 12 | 202688_at | Tumor necrosis factor (lig) superfamily, member 10 (2 probesets) | TNFSF10 (TRAIL) | m1 | Up |
| | 202687_s_at | | | m1 | |
| 13 | 202864_s_at | SP100 nuclear antigen | SP100 | m1 | Up |
| 14 | 210164_at | Granzyme B | GZMB | m1 | Up |
| 15 | 214453_s_at | Interferon-induced protein 44 | IFI44 | y1 | Up |
| 16 | 227510_x_at | PRO1073 protein (histone deacetylase 3) | PR01073 | y1 | Up/Do |
| 17 | 202430_s_at | Phospholipid scramblase 1 | PLSCR1 | y1 | Up |
